(19) Europäisches Patentamt
European Patent Office
Office européen des brevets

(11) Veröffentlichungsnummer : **0 183 149**
**B1**

(12) # EUROPÄISCHE PATENTSCHRIFT

(45) Veröffentlichungstag der Patentschrift : **15.06.88**

(51) Int. Cl.⁴ : **C 07 C 67/31**, C 07 C 69/716

(21) Anmeldenummer : **85114566.4**

(22) Anmeldetag : **16.11.85**

(54) **Verfahren zur Herstellung von 4-Acyloxy-3-oxo-butter-säureestern.**

(30) Priorität : **30.11.84 DE 3443678**

(43) Veröffentlichungstag der Anmeldung : **04.06.86 Patentblatt 86/23**

(45) Bekanntmachung des Hinweises auf die Patenterteilung : **15.06.88 Patentblatt 88/24**

(84) Benannte Vertragsstaaten : **AT BE CH DE FR GB IT LI NL SE**

(56) Entgegenhaltungen : **EP-A- 0 076 379**

(73) Patentinhaber : **BAYER AG**
**Konzernverwaltung RP Patentabteilung**
**D-5090 Leverkusen 1 Bayerwerk (DE)**

(72) Erfinder : **Stubbe, Mathias, Dr.**
**Zur Waldesruh 100**
**D-5600 Wuppertal 11 (DE)**

**EP 0 183 149 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein neues Verfahren zur Herstellung von teilweise bekannten 4-Acyloxy-3-oxobuttersäureestern, die zur Herstellung von pharmazeutischen Wirkstoffen verwendet werden können.

Alle bisher bekannten Verfahren sind entweder aufwendig oder sicherheitstechnisch und ökologisch nicht unbedenklich (vgl. I. Ratusky, F. Sorm, Chem. listy 51, 1091-1100 (1957) ; J. I. De Graw, Tetrahedron 28, 967-972 (1972) ; A. L. Veresh chogin, A. A. Semenow, Zh. Org. Khim 18, 1772/3 (1982)).

Es wurde nun gefunden, daß man die 4-Acyloxy-3-oxobuttersäureester der allgemeinen Formel (I)

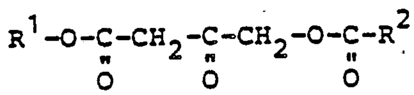

(I)

in welcher

$R^1$ für gerades oder verzweigtes Alkyl mit bis zu 4 C-Atomen, bevorzugt für Methyl und Ethyl und

$R^2$ für gerades oder verzweigtes Alkyl mit bis zu 4 C-Atomen (gegebenenfalls substituiert durch Halogen, Hydroxy, Alkoxy oder Phenyl), bevorzugt mit bis zu 2 C-Atomen, besonders bevorzugt für Methyl oder für Aryl, bevorzugt für Phenyl steht,

erhält, wenn man 4-Halogenbuttersäureester der allgemeinen Formel (II)

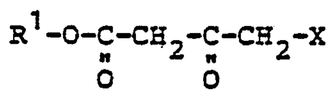

(II)

in welcher

$R^1$ die oben angegebene Bedeutung hat und

X für Chlor oder Brom, bevorzugt für Chlor steht

mit Carbonsäuren der allgemeinen Formel (III)

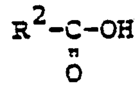

(III)

in welcher $R^2$ die oben angegebene Bedeutung hat in Gegenwart von Säurefängern in aprotischen organischen Lösungsmitteln umsetzt.

Das erfindungsgemäße Verfahren liefert überraschenderweise nicht nur bei Verwendung der Bromderivate, sondern auch der Chlorderivate der Verbindung (II) das Endprodukt in guten Ausbeuten. Außerdem ist die Aufarbeitung und Reinigung der Endprodukte technisch leicht durchzuführen.

Die erfindungsgemäß erhältlichen Verbindungen der Formel (I) eignen sich insbesondere zur Herstellung von 1,4-Dihydropyridinen die in 2- und 3-Position einen Lactonring tragen. Die Herstellung erfolgt z. B. durch Umsetzung von Verbindungen der Formel (I) mit Aldehyden gegebenenfalls nach Isolierung der entstehenden Ylidenverbindungen, und mit Aminocrotonsäureestern zu den entsprechenden Dihydropyridinen (vergl. DE-OS 3 206 671).

Verwendet man als Ausgangsstoffe 4-Chlor-3-oxo-buttersäuremethylester und Essigsäure, so kann der Reaktionsverlauf durch folgendes Schema verdeutlicht werden :

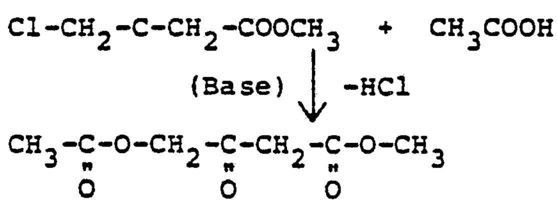


Die Ausgangsprodukte sind bekannt oder können nach bekannten Verfahren hergestellt werden.

Als Lösungsmittel kommen alle protischen und aprotischen organischen Lösungsmittel in Frage. Hierzu gehören halogenierte Kohlenwasserstoffe wie Dichlor-, Trichlormethan, Trichlorethylen, Ether wie Diethylether, Dioxan, Tetrahydrofuran oder Glykolmono-, Glykoldimethylether, Kohlenwasserstoffe wie Toluol oder Xylol, sekundäre und tertiäre Alkohole wie Isopropanol, 2-Butanol und tert.-Butanol, Dimethylformamid, Methylisobutylketon oder Acetonitril. Bevorzugte Lösungsmittel sind Toluol, Dimethylformamid oder Methylisobutylketon.

Als Säurefänger kommen die üblichen organischen Basen in Frage. Hierzu gehören tertiäre und sekundäre Alkylamine, Pyridin oder Piperidin. Besonders bevorzugt ist Triethylamin.

Die Umsetzung wird bei Temperaturen von 0-170 °C, bevorzugt bei Temperaturen von 60-120 °C

durchgeführt. Gegebenenfalls hat es sich als günstig erwiesen beim Siedepunkt des verwendeten Lösungsmittels zu arbeiten.

Die Umsetzung kann bei normalem, aber auch bei erhöhtem Druck durchgeführt werden. Im allgemeinen arbeitet man bei Normaldruck.

Bei der Durchführung des erfindungsgemäßen Verfahrens setzt man pro Mol der Halogenverbindung der Formel (II) 1-5 Mol, bevorzugt 1-2,5 Mol der Carbonsäure der Formel (III) ein. Pro Mol der Carbonsäure der Formel (III) wiederum werden 0,5-1,5 Mol Säurefänger eingesetzt. Gegebenenfalls ist es auch möglich, die Ammoniumsalze der Carbonsäuren zu verwenden.

Die Reinigung der Produkte kann sehr einfach durch Extrahieren der wasserlöslichen Anteile und nachfolgende Destillation oder Kristallisation erfolgen.

Beispiel 1 : 4-Acetoxy-3-oxobuttersäureethylester

Eine Lösung von 1,204 l (21,0 mol) Eisessig und 2,786 l (20,0 mol) Triethylamin in 8,75 l Methylisobutylketon wird unter Rückfluß erhitzt und in der Siedehitze eine Lösung von 1,498 l (11,2 mol) 4-Chlor-3-oxobuttersäure-ethylester in 1,75 l Methylisobutylketon innerhalb von 20 Minuten zugegeben. Nach beendeter Zugabe wird weitere 30 Minuten unter Rückfluß erhitzt, auf ca. 5 °C abgekühlt und ausgefallene Salze abfiltriert. Der Filterrückstand wird mit 4,5 l Methylisobutylketon gewaschen. Die vereinigten filtrate werden im Vakuum eingeengt, zum Rückstand werden zunächst 2 l Trinkwasser gegeben, dann 4 l einer 9 %igen Natriumhydrogencarbonat-Lösung. Die Suspension wird dreimal mit je 4 l Essigester extrahiert, die vereinigten Extrakte werden über 2 kg Natriumsulfat getrocknet, abfiltriert und bei 40 °C Badtemperatur am Rotationsverdampfer eingeengt.

Der Rückstand wird fraktioniert destilliert.

Ausbeute : 885 g (42 % der Theorie)

Sdp. : 84-86 °C/0,4 torr

Beispiel 2 : 4-Acetoxy-3-oxobuttersäureethylester

Nach der in Beispiel 1 beschriebenen Arbeitsweise werden 71 ml 4-Chlor-3-oxobuttersäureethylester mit 57 ml Essigsäure und 132 ml Triethylamin in 1 l Dimethylformamid umgesetzt.

Ausbeute : 43,2 g (43,3 % der Theorie)

Beispiel 3 : 4-Acetoxy-3-oxobuttersäureethylester

Nach der in Beispiel 1 beschriebenen Arbeitsweise werden 71 ml 4-Chlor-3-oxobuttersäureethylester mit 57 ml Essigsäure und 132 ml Triethylamin in 1 l 2-Butanol umgesetzt.

Ausbeute : 42,4 g (42,5 % der Theorie)

Beispiel 4 : 4-Propionyloxy-3-oxobuttersäureethylester

In einer siedenden Lösung von 73 ml Propionsäure und 132 ml Triethylamin in 833 ml Toluol wird innerhalb von 30 Minuten eine Lösung von 71 ml 4-Chlor-3-oxobuttersäureethylester in 165 ml Toluol zugegeben. Es wird 90 Minuten unter Rückfluß gekocht, dann abgekühlt, das ausgefallene Triethylaminhydrochlorid abgesaugt, zweimal mit je 300 ml Wasser extrahiert und das Lösungsmittel im Vakuum abdestilliert. Der Rückstand wird im Hochvakuum fraktioniert destilliert.

Ausbeute : 48,0 g (44,8 % der Theorie)

$C_9H_{41}O_5$ (202,2)

Ber. : C 53,5 H 6,6 O 39,6

Gef. : C 53,2 H 6,9 O 39,8

Beispiel 5 : 4-Benzyloxy-3-oxobuttersäureethylester

Nach der in Beispiel 4 beschriebenen Arbeitsweise werden 71 ml 4-Chlor-3-oxobuttersäureethylester mit 121,4 g Benzoesäure und 132 ml Triethylamin in 1 l Toluol umgesetzt. Der Rückstand nach destillativer Entfernung des Lösungsmittels ist praktisch reines Produkt.

Ausbeute : 118,3 g (89 % der Theorie)

$C_{13}H_{14}O_5$ (250,3)

Ber. : C 62,4 H 5,6 O 32,0

Gef. : C 62,7 H 5,5 O 31,8

$^1$H-NMR (250 MHz, $CDCl_3$) $\delta$ = 1,30 (t, 3H ; $CH_2$—$CH_3$)
3,52 (S, 2H ; 2-H), 4,24 (q, 2H ; O—$CH_2$—$CH_3$),
5,60 (S, 2H ; 4-H), 7,52 (t, 2H ; 3',5'-H),
7,66 (t, 1H ; 4'-H), 8,14 (d, 2H ; 2',6'-H)

Beispiel 6 : 4-(Methoxyacetoxy)-3-oxobuttersäureethylester

Nach der in Beispiel 4 beschriebenen Arbeitsweise werden 71 ml 4-Chlor-3-oxobuttersäureethylester mit 89,5 g Methoxyessigsäure und 132 ml Triethylamin in 1 l Toluol umgesetzt. Das Produkt wird durch Kugelrohrdestillation gereinigt.

Ausbeute : 44,3 g (38,3 % der Theorie)

$C_9H_{14}O_6$ (218,2)

Ber. : C 49,5 H 6,5 O 44,0

Gef. : C 49,8 H 6,6 O 43,7

$^1$H-NMR (250 MHz, $COCl_3$) $\delta$ = 1,27 (t, 3H, O—$CH_2$—$CH_3$) ; 3,47 (S, 3H, O—$CH_3$), 3,50 (S, 2H, 2-H), 4,14 (S, 2H, CO—$CH_2$—O—$CH_3$), 4,24 (q, 2H, O—$CH_2$—$CH_3$), 4,90 (S, 2H, 4-H).

**Patentansprüche**

1. Verfahren zur Herstellung von 4-Acyloxy-3-oxo-buttersäureestern der allgemeinen Formel (I)

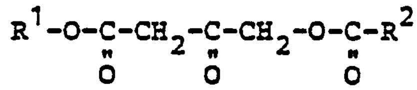

(I)

in welcher

$R^1$ für geradkettiges oder verzweigtes Alkyl mit bis zu 4 C-Atomen steht, und

$R^2$ für geradkettiges oder verzweigtes, gegebenenfalls durch Halogen, Hydroxy, Alkoxy oder Phenyl substituiertes Alkyl mit bis zu 4 C-Atomen oder für Aryl steht,

dadurch gekennzeichnet, daß man 4-Halogenbuttersäureester der allgemeinen Formel (II)

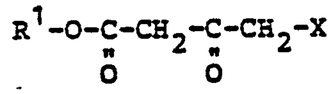

(II)

in welcher

$R^1$ die oben angegebene Bedeutung hat, und

X für Chlor oder Brom steht

mit Carbonsäuren der allgemeinen Formel (III)

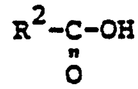

(III)

in welcher $R^2$ die oben angegebene Bedeutung hat, in Gegenwart von Säurefängern in protischen oder aprotischen organischen Lösungsmitteln bei Temperaturen zwischen 0-170 °C umsetzt.

2. Verfahren zur Herstellung von 4-Acyloxy-3-oxo-buttersäureestern der allgemeinen Formel (I) gemäß Anspruch 1

in welcher

$R^1$ für Methyl oder Ethyl steht und

$R^2$ für geradkettiges oder verzweigtes Alkyl mit bis zu 4 C-Atomen steht, welches gegebenenfalls substituiert ist durch Fluor, Chlor oder Brom, Hydroxy, Alkoxy mit 1 bis 2 C-Atomen oder Phenyl oder für Phenyl steht,

dadurch gekenzeichnet, daß man 4-Halogenbuttersäureester der allgemeinen Formel (II) mit Carbonsäureestern der allgemeinen Formel (III) gemäß Anspruch 1 bei Temperaturen von 60-120 °C durchführt.

3. Verfahren gemäß den Ansprüchen 1 und 2, dadurch gekennzeichnet, daß man als organische Lösungsmittel halogenierte Kohlenwasserstoffe, Ether, sekundäre und tertiäre Alkohole, Kohlenwasserstoffe, Dimethylformamid oder Methylisobutylketon oder Gemische hiervon einsetzt.

4. Verfahren gemäß Ansprüchen 1, 2 und 3, dadurch gekennzeichnet, daß man als Säurefänger organische Basen aus der Gruppe sekundärer und tertiärer Alkylamine, Pyridin, Piperidin oder Ammoniumsalz von Carbonsäuren einsetzt.

5. Verfahren gemäß den Ansprüchen 1, 2, 3 und 4, dadurch gekennzeichnet, daß man pro Mol der Halogenverbindung der Formel (II) 1-5 Mol der Carbonsäure der Formel (III) und pro Mol der Carbonsäure der Formel (III) 0,5-1,5 Mol Säurefänger einsetzt.

6. Verwendung von Verbindungen der allgemeinen Formel (I) erhältlich nach den Ansprüchen 1 bis 5 zur Herstellung von pharmazeutischen Wirkstoffen.

7. Verwendung gemäß Anspruch 6 zur Herstellung von Dihydropyridinen mit Lactonstruktur der Substituenten in 2- und 3-Position durch cyclisierende Umsetzung mit Aldehyden und Aminocrotonsäureestern.

8. Verwendung von Verbindungen der Formel I gemäß Ansprüchen 6 und 7 durch Umsetzung mit

Aldehyden, Isolierung der hierbei entstehenden Ylidenverbindungen und deren weitere Umsetzung mit Aminocrotonsäureestern.

## Claims

1. Process for the preparation of 4-acyloxy-3-oxobutyric acid esters of the general formula (I)

$$R^1-O-\underset{\underset{O}{\|}}{C}-CH_2-\underset{\underset{O}{\|}}{C}-CH_2-O-\underset{\underset{O}{\|}}{C}-R^2 \qquad (I)$$

in which

$R^1$ represents straight-chain or branched alkyl with up to 4 C atoms and

$R^2$ represents straight-chain or branched alkyl which has up to 4 C atoms and is optionally substituted by halogen, hydroxyl, alkoxy or phenyl, or represents aryl,

characterised in that 4-halogenobutyric acid esters of the general formula (II)

$$R^1-O-\underset{\underset{O}{\|}}{C}-CH_2-\underset{\underset{O}{\|}}{C}-CH_2-X \qquad (II)$$

in which

$R^1$ has the abovementioned meaning and

X represents chlorine or bromine

are reacted with carboxylic acids of the general formula (III)

$$R^2-\underset{\underset{O}{\|}}{C}-OH \qquad (III)$$

in which $R^2$ has the abovementioned meaning, in the presence of acid-trapping agents in protic or aprotic organic solvents at temperatures between 0 and 170 °C.

2. Process for the preparation of 4-acyloxy-3-oxo-butyric acid esters of the general formula (I) according to Claim 1,

in which

$R^1$ represents methyl or ethyl and

$R^2$ represents straight-chain or branched alkyl with up to 4 C atoms, which is optionally substituted by fluorine, chlorine or bromine, hydroxyl, alkoxy with 1 or 2 C atoms or phenyl, or represents phenyl,

characterised in that 4-halogenobutyric acid esters of the general formula (II) are carried out with carboxylic acid esters of the general formula (III) according to Claim 1 at temperatures of 60-120 °C.

3. Process according to Claims 1 and 2, characterised in that halogenated hydrocarbons, ethers, secondary and tertiary alcohols, hydrocarbons, dimethylformamide or methyl isobutyl ketone or mixtures thereof are employed as the organic solvents.

4. Process according to Claims 1, 2 and 3, characterised in that organic bases from the group comprising secondary and tertiary alkylamines, pyridine, piperidine or an ammonium salt of carboxylic acids are employed as the acid-trapping agents.

5. Process according to Claims 1, 2, 3 and 4, characterised in that 1-5 moles of the carboxylic acid of the formula (III) are employed per mole of the halogen compound of the formula (II), and 0.5-1.5 moles of acid-trapping agent are employed per mole of the carboxylic acid of the formula (III).

6. Use of compounds of the general formula (I) obtainable according to Claims 1 to 5 for the preparation of pharmaceutical active compounds.

7. Use according to Claim 6 for the preparation of dihydropyridines with a lactone structure of the substituents in the 2- and 3-position, by cyclising reaction with aldehydes and aminocrotonic acid esters.

8. Use of compounds of the formula I according to Claims 6 and 7 by reaction with aldehydes, isolation of the ylidene compounds thereby formed and further reaction thereof with aminocrotonic acid esters.

## Revendications

1. Procédé de production d'esters d'acides 4-acyloxy-3-oxobutyriques de formule générale (I)

$$R^1-O-\underset{\underset{O}{\|}}{C}-CH_2-\underset{\underset{O}{\|}}{C}-CH_2-O-\underset{\underset{O}{\|}}{C}-R^2 \qquad (I)$$

dans laquelle

R¹ est un groupe alkyle à chaîne droite ou à chaîne ramifiée ayant jusqu'à 4 atomes de carbone, et

R² est un groupe alkyle à chaîne droite ou ramifiée ayant jusqu'à 4 atomes de carbone, éventuellement substitué par un halogène, un radical hydroxy, alkoxy ou phényle, ou est un reste aryle, caractérisé en ce qu'on fait réagir des esters d'acides 4-halogénobutyriques de formule générale (II)

$$R^1-O-\underset{\underset{O}{\|}}{C}-CH_2-\underset{\underset{O}{\|}}{C}-CH_2-X \qquad (II)$$

dans laquelle

$R^1$ a la définition indiquée ci-dessus, et

X est le chlore ou le brome

avec des acides carboxyliques de formule générale (III)

$$R^2-\underset{\underset{O}{\|}}{C}-OH \qquad (III)$$

dans laquelle $R^2$ a la définition indiquée ci-dessus, en présence d'accepteurs d'acides dans des solvants organiques protiques ou aprotiques à des températures comprises entre 0 et 170 °C.

2. Procédé de production d'esters d'acides 4-acyloxy-3-oxobutyriques de formule générale (I) suivant la revendication 1

dans laquelle

$R^1$ est un groupe méthyle ou éthyle et

$R^2$ est un groupe alkyle à chaîne droite ou à chaîne ramifiée ayant jusqu'à 4 atomes de carbone, qui est éventuellement substitué par du fluor, du chlore ou du brome, un radical hydroxy, alkoxy ayant 1 ou 2 atomes de carbone ou phényle, ou représente un reste phényle,

caractérisé en ce qu'on fait réagir des esters d'acides 4-halogénobutyriques de formule générale (II) avec des esters d'acides carboxyliques de formule générale (III) suivant la revendication 1 à des températures de 60 à 120 °C.

3. Procédé suivant les revendications 1 et 2, caractérisé en ce qu'on utilise comme solvants organiques des hydrocarbures halogénés, des éthers, des alcools secondaires et tertiaires, des hydrocarbures, le diméthyl-formamide et la méthylisobutylcétone ou des mélanges de ces solvants.

4. Procédé suivant les revendications 1, 2 et 3, caractérisé en ce qu'on utilise comme accepteurs d'acides des bases organiques du groupe comprenant des alkylamines secondaires et tertiaires, la pyridine, la pipéridine ou un sel d'ammonium d'acides carboxyliques.

5. Procédé suivant les revendications 1, 2, 3 et 4, caractérisé en ce qu'on utilise, par mole de composé halogéné de formule (II), 1 à 5 moles d'acide carboxylique de formule (III) et, par mole d'acide carboxylique de formule (III), 0,5 à 1,5 mole d'accepteur d'acide.

6. Utilisation de composés de formule générale (I) pouvant être obtenus selon les revendications 1 à 5 pour la préparation de substances actives pharmaceutiques.

7. Utilisation suivant la revendication 6 pour la préparation de dihydropyridines à structure de lactone des substituants en positions 2 et 3 par réaction cyclisante avec des aldéhydes et des esters d'acide aminocrotonique.

8. Utilisation de composés de formule I suivant les revendications 6 et 7 par réaction avec des aldéhydes, isolement des composés ylidéniques ainsi formés puis réaction de ce composé avec des esters d'acide aminocrotonique.